# EUROPEAN PATENT SPECIFICATION

(11) **EP 3 052 026 B1**
(45) Date of publication and mention of the grant of the patent: **19.09.2018**
(21) Application number: 14792618.2
(22) Date of filing: 26.09.2014
(51) Int. Cl.: A61B 10/02, G01N 33/536, B82Y 5/00, B82Y 25/00, G01N 33/543

(54) **SYSTEM FOR ISOLATION OF CIRCULATING CELLS FROM PERIPHERAL BLOOD**
SYSTEM ZUR ISOLIERUNG VON ZIRKULIERENDEN ZELLEN AUS PERIPHEREM BLUT
SYSTÈME POUR L'ISOLEMENT DE CELLULES CIRCULANTES À PARTIR DE SANG PÉRIPHÉRIQUE

(30) Priority: 01.10.2013 SK 922013
(43) Date of publication of application: 10.08.2016
(73) Proprietor: Ustav Stavebnictva A Architektury Sav, 84503 Bratislava 45 (SK); Univerzita Komenskeho V Bratislave, 818 06 Bratislava (SK); Centrum Vedecko-Technickych Informacii SR (CVTI SR), 811 04 Bratislava (SK)
(72) Inventor: KOCIFAJ, Miroslav, 841 07 Bratislava (SK); MEGO, Michal, 833 01 Bratislava (SK)
(74) Representative: Herrmann, Jochen
(86) International application number: PCT/SK2014/000020
(87) International publication number: WO 2015/050507

(56) References cited:
- US-A1- 2013 197 296
- US-B1- 7 309 316
- SAUCEDO-ZENI N ET AL: "A novel method for the in vivo isolation of circulating tumor cells from peripheral blood of cancer patients using a functionalized and structured medical wire", INTERNATIONAL JOURNAL OF ONCOLOGY, DEMETRIOS A. SPANDIDOS ED. & PUB, GR, vol. 41, no. 4, 1 October 2012 (2012-10-01), pages 1241-1250, XP002731287, ISSN: 1019-6439, DOI: 10.3892/IJO.2012.1557 [retrieved on 2012-07-16]

## Description

### Field of the invention

The proposed solution refers to a system for isolation of circulating blood cells from peripheral blood by means of magnetic nanoparticles covered with monoclonal antibodies.

### Description of the related art

Metastases of solid tumors (with the exception of those of embryonic cells and some lymphomas) are usually incurable (Greenberg, 1996); conventional chemotherapy has only palliative effect on almost all patients, exceptions are only individual. Recent progress shows that the cancer stem cells play an important role in the progression of metastases (Kakarala, 2008). Cancer stem cells were isolated from the human tumors including hematological malignancies, glioblastoma, breast carcinoma, while these cells have intrinsic resistance towards chemotherapy and radiotherapy (Liu, 2006, Ishikawa, 2007, Li, 2008). The identification of intratumor and intertumor heterogeneities has shown the need of more exact tumor phenotype definition with individual patients and subsequently more effective treatment.

The new approach is based on the understanding of mechanisms leading to tumor progression with further possibilities of targeted manipulation of this process. Tumor dissemination involves circulating tumor cells (CTC) that are the key step in metastasis formation. CTC are very rare cells surrounded by milliards of blood cells in the bloodstream. Recent progress in detection methods allowed for their reproducible identification and further characterization of CTC in peripheral blood (Mego, 2010). Various strategies are employed to achieve this, including the use of morphological and physical properties such as dimensions and mass, or detection of expression of specific markers. CTC determination of carcinoma of epithelial cells is based on detection of epithelial markers, such as cytokeratin (protein of cytoskeleton present in epithelial cells) or EpCaM (epithelial cell adhesive molecule) and based on the absence of common leukocyte markers (CD45) and/ or in presence of tumor-specific antigens (e.g. MUC1 and HER2) (Mego, 2010).

CTC are markers of so called tumor self-seeding (Kim, 2009), and it is assumed that patients with fast growing tumors have a higher count of CTC in the peripheral blood. Owing to low count of CTC in blood is our biological and clinical knowledge of CTC highly dependent on parameters of isolation and used technology, which usually entails enrichment steps towards improved detection rate.

CTC detection of patients with solid tumors including breast carcinoma, prostate and colorectal cancer gained considerable attention in the course of last 10 years (Cristofanilli, 2004, De Bono, 2008, Cohen, 2008). In spite of recent technological advances we are still only in the beginning of understanding the processes related to the role of CTC in tumor progression and dissemination. The possibility of sequential blood sample collection for monitoring of systematic treatment in real time has opened a new way towards development of surrogate biomarkers for improved clinical decision making. In addition to that the complex molecular characterization of CTC should provide important insight to biology of metastases. Some findings of phenotypic screening have proven that at least some CTC have properties of tumor stem cells. This data suggests the possibility to assess the effect of antitumor medication to tumor stem cells using the phenotype definition of CTC (Mego, 2010).

Several methods for detection and isolation of blood cells of various types presently exist, e.g. documents US 2009/0220979 A1, US 2012/0315639 A1, US 2012/0252088 A1 and WO 2012/122627 A1 describe such solutions. The disadvantage of the known solutions is in very small resulting quantity of isolated blood cells that does not allow for better cell characterization. Further, systems for isolation of circulating blood cells from peripheral blood by means of magnetic nanoparticles covered with monoclonal antibodies are known from US 2013/01972296 A1 and US 7,309,316 B1. Finally, Saucedo-Zeni, N. et al.: A Novel Method for the In Vivo Isolation of Circulating Tumor Cells from Peripheral Blood of Cancer Patients Using a Functionalized and Structured Medical Wire, International Journal of Oncology, Demetrious A. Spandidos Ed. & Pub., GR, Vol. 41, No. 4, 1st October 2012, pages 1241-1250 describes another possibility of isolating circulating blood cells from peripheral blood.

### Summary of the invention

The presented solution to a large extent addresses the shortcomings of the known methods. The present invention proposes a system for isolation of circulating cells from peripheral blood comprising:
- a vein cannula adapted for slowly injecting the nanoparticles into a patient's vein,
- magnetic nanoparticles covered with monoclonal antibodies,
- magnets located outside the patient's body and adapted for controlling the position of the injected nanoparticles in the vein,
- the magnets being further adapted for moving the nanoparticles closer to the vein cannula with the movement of the magnets after exposure of the nanoparticles to circulating blood cells and after the blood cells are captured by bonding to the antibodies on the surface of the nanoparticles,
- a wire with a magnetic core adapted for being inserted into the vein through the cannula,
- the wire further being adapted for attracting the nanoparticles after the magnets are removed or switched off, and
- a tube with a medium adapted for receiving the wire with blood cells after its removal from the cannula and in which tube the nanoparticles with the bound blood cells are released after removal of the magnetic core of the wire.

Its advantage in comparison with existing solutions resides in the ability to isolate more CTC (or other cells present in peripheral blood) than other methods. The isolation is done in blood not leaving the bloodstream and needs no dilution as other methods require. Nanoparticles floating in the blood are exposed to CTC of the whole volume of blood. This system is thus capable of isolating the CTC not only from limited volume of blood (as is the case of other methods) but from the whole volume of blood.

Compared to existing methods for isolation of CTC this system allows for repeated isolation of CTC from the whole volume of blood without blood leaving the bloodstream or sampling it. Compared to systems used e.g. for separation of hematopoietic stem cells it is a considerable advantage that blood needs not to leave the bloodstream for the isolation of target cells and thus no anticoagulant drugs need to be administered to the patient, which decreases the risk of complications and undesired effects on the patient.

Preferred embodiments of the invention are the subject matter of the dependent claims.

The isolation of circulating cells from the peripheral blood is according to this solution done so that the magnetic nanoparticles covered with monoclonal antibodies are slowly injected into a patient's vein through an intravenous cannula (peripheral venous catheter or central venous catheter). The position of the nanoparticles is controlled by magnets (stationary or permanent) with an intensity 0,2 - 1 T, while the movement of nanoparticles is limited to a defined part of the body. The nanoparticles are exposed to circulating blood cells and using the bonding of antibodies capture them on their surface. The nanoparticles are then moved closer to the intravenous cannula through the movement of magnets (stationary or permanent) and subsequently a special wire with a magnetic core is inserted through the cannula and the magnets (stationary or permanent) are removed or switched off. The nanoparticles are attracted by the wire and they are removed from the cannula together with the wire and placed in a tube with medium. The magnetic core of the wire is removed and the nanoparticles with captured blood cells are released into the medium. Individual steps of this method may be repeated as needed (e.g. in case of low count of captured cells). The wire is made of magnetic material with transverse magnetization towards the wire axis, while the preferred length of the wire is 3 to 30 cm and preferred thickness of the wire is 1 to 3 mm. The magnetic core of the wire is placed in a flexible non-magnetic plastic cover and serves to hold the particles without an external magnetic field. The wire must fulfill following properties: its magnetic power is 0.2 to 1 T, it must be highly flexible (e.g. with the core made of magnetic powder) and covered with a sterile plastic. The wire should be transversely magnetized, which allows for more efficient collection of magnetic particles that are preferably attracted towards a stronger pole. This solution provides an advantage in respect of external magnets, a competing effect of which can thus be eliminated. Magnetic materials for such wire are for instance supramagnetic samarium or neodymium alloys or magnetic ferrite alloys. This method of isolation of circulating cells from peripheral blood can be used for various types of blood cells, e.g. for circulating tumor cells, hematopoietic stem cells usable for bone marrow transplant, mesenchymal stem cells or any circulating cells depending on monoclonal antibodies used.

### Brief description of the drawings

The proposed solution is further clarified by the figures:
- Fig. 1: depicts magnetic nanoparticles (A) inserted in the blood;
- Fig. 2: depicts the special wire inserted through the cannula into the bloodstream;
- Fig. 3: depicts the special wire covered with nanoparticles and captured CTC;
- Fig. 4: depicts how the nanoparticles (A) with CTC are released into a medium for further analysis.

### Detailed description of the invention

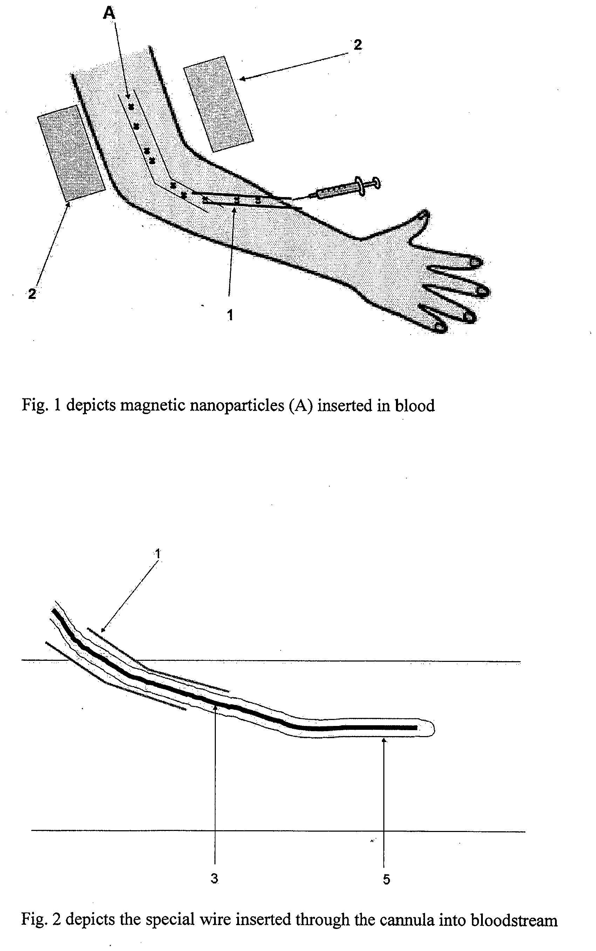

The system is based on the isolation of blood cells using the magnetic nanoparticles that are covered with monoclonal antibodies. Magnetic nanoparticles covered with monoclonal antibodies against common antigen epitel (EpCAM, CD326) are slowly injected into a patient's vein (forearm or other suitable vein) through a vein cannula 1 (Fig. 1). The position of the nanoparticles is controlled by large magnets 2 and their movement is limited to e.g. the shoulder area or other body parts. Electromagnets or permanent large magnets 2 may be fixed to the respective body part, thus not limiting the patient to sitting or laying, but allowing him to perform ordinary activities during the procedure.

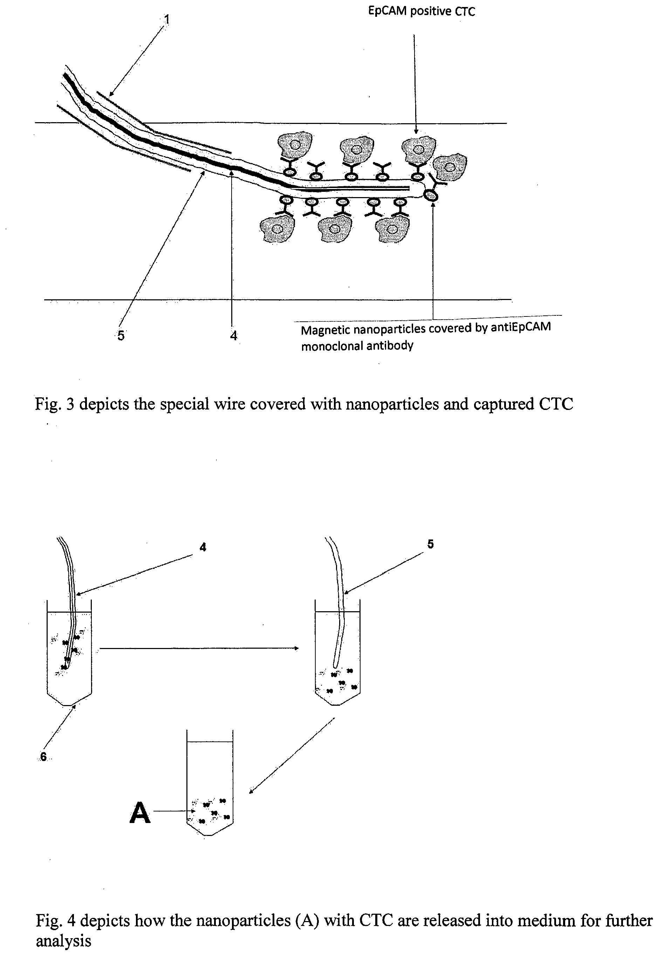

The nanoparticles are exposed to circulating tumor cells, and they are captured by bonding to antibodies EpCAM on the nanoparticles surface. Conductor wire 3 is used to remove the nanoparticles with bound CTC from the bloodstream. After incubation the nanoparticles are moved closer to the vein cannula 1 by the movement of the large magnets 2. Then the conductor wire 3 is inserted through the cannula 1 into the bloodstream (Fig. 2). If the permanent magnets 2 are used, these are removed (optionally the electromagnets are switched off) and the nanoparticles are attracted to the magnetic conductor wire 3 (Fig. 3).

The magnetic conductor wire 3 covered with the nanoparticles with bound CTC is subsequently removed from the cannula 1. The wire 3 is introduced into a tube 6 with a suitable medium. The magnetic core 4 is removed and nanoparticles with bound CTC are released into the medium for further analysis (Fig. 4). Optionally it is possible to place a permanent magnet under the tube 6 that will stabilize particles and simplify the manipulation as described above. Wire 3 is built of magnetic material (such as samarium or neodymium based supramagnetic alloys or magnetic alloys of ferrite) with magnetic power 0.2 - 1 T, that secures holding of particles without external magnetic field. The preferred length of the wire is 3 to 30 cm and the preferred thickness of the wire is 1 to 3 mm. The wire 3 is placed in a sterile flexible plastic cover 5. The wire 3 is transversely magnetized, which allows for more efficient collection of magnetic particles that are preferably attracted towards a stronger pole. This solution provides an advantage in respect of external magnets, the competing effect of which can thus be eliminated.

### Industrial applicability

The system may be used in medicine for isolation of various types of blood cells depending on the monoclonal antibodies used. The solution will be useful in the oncological research, for isolation of dendritic cells for development of tumor vaccines, or mesenchymal stem cells with the application in the regenerative medicine and/ or transplantation of stem cells. The use of this method in the area of oncological diseases will allow for better characterization of CTC (circulating tumor cells), identification of therapeutic targets, performing the molecular profiling of tumors that are disseminated via CTC, that will subsequently allow for better analysis of mechanism of resistance and/or development of antitumor vaccine from predominant CTC clones.

## Claims

1. System for isolation of circulating cells from peripheral blood comprising:
- a vein cannula (1) adapted for slowly injecting the nanoparticles into a patient's vein,
- magnetic nanoparticles covered with monoclonal antibodies,
- magnets (2) located outside the patient's body and adapted for controlling the position of the injected nanoparticles in the vein,
- the magnets (2) being further adapted for moving the nanoparticles closer to the vein cannula (1) with the movement of the magnets (2) after exposure of the nanoparticles to circulating blood cells and after the blood cells are captured by bonding to the antibodies on the surface of the nanoparticles,
- a wire (3) with a magnetic core (4) adapted for being inserted into the vein through the cannula (1),
- the wire (3) further being adapted for attracting the nanoparticles after the magnets (2) are removed or switched off, and
- a tube (6) with a medium adapted for receiving the wire (3) with blood cells after its removal from the cannula (1) and in which tube (6) the nanoparticles with the bound blood cells are released after removal of the magnetic core (4) of the wire (3).

2. System according to claim 1, **characterized in that** the vein cannula (1) is a peripheral cannula or a central venous catheter.

3. System according to claim 1 or 2, **characterized in that** the magnets (2) that control the position of nanoparticles and are stationary electromagnets or permanent magnets with an intensity of 0.2 - 1 T.

4. System according to one of the preceding claims, **characterized in that** the wire (3) is made of magnetic material with transverse magnetization towards the wire axis.

5. System according to one of the preceding claims, **characterized in that** the magnetic power of the wire (3) is 0.2 to 1 T.

6. System according to one of the preceding claims, **characterized in that** the wire (3) is highly flexible.

7. System according to one of the preceding claims, **characterized in that** the length of the wire (3) is in the range of 3 to 30 cm and a preferred thickness of the wire (3) is 1 to 3 mm.

8. System according to one of the preceding claims, **characterized in that** the magnetic core (4) of the wire (3) is made of magnetic powder.

9. System according to one of the preceding claims, **characterized in that** the magnetic core (4) of the wire (3) is placed in a flexible non-magnetic cover (5) made of sterile plastic.

10. System according to one of the preceding claims, **characterized in that** the system is adapted for isolation of circulating tumor cells, hematopoieticstem cells, mesenchymal stem cells, or any other circulating cells depending on the monoclonal antibodies used.

## Patentansprüche

1. System zur Isolierung von zirkulierenden Zellen aus peripherem Blut, umfassend:
- eine Venenkanüle (1), die ausgestaltet ist, um die Nanopartikel langsam in die Vene eines Patienten zu injizieren,
- magnetische Nanopartikel, die mit monoklonalen Antikörpern bedeckt sind,
- Magnete (2), die sich außerhalb des Körpers des Patienten befinden und zum Steuern der Position der injizierten Nanopartikel in der Vene ausgestaltet sind,
- wobei die Magnete (2) ferner dafür ausgestaltet sind, die Nanopartikel, nachdem die Nanopartikel den zirkulierenden Blutzellen ausgesetzt waren und nachdem die Blutzellen durch Binden an die Antikörper an der Oberfläche der Nanopartikel eingefangen sind, mit der Bewegung der Magnete (2) näher an die Venenkanüle (1) zu bewegen,
- einen Draht (3) mit einem Magnetkern (4), der dafür ausgestaltet ist, durch die Kanüle (1) in die Vene eingeführt zu werden,
- wobei der Draht (3) ferner dazu ausgestaltet ist, die Nanopartikel anzuziehen, nachdem die Magnete (2) entfernt oder ausgeschaltet worden sind, und
- ein Röhrchen (6) mit einem Medium, das zur Aufnahme des Drahtes (3) mit Blutzellen nach dessen Entfernung aus der Kanüle (1) ausgestaltet ist und wobei in dem Röhrchen (6) die Nanopartikel mit den gebundenen Blutzellen nach Entfernung des Magnetkerns (4) des Drahtes (3) freigesetzt werden.

2. System nach Anspruch 1, **dadurch gekennzeichnet, dass** die Venenkanüle (1) eine periphere Kanüle oder ein zentralvenöser Katheter ist.

3. System nach Anspruch 1 oder 2, **dadurch gekennzeichnet, dass** die Magnete (2) die Position von Nanopartikeln steuern und stationäre Elektromagnete oder Permanentmagnete mit einer Intensität von 0,2-1 T sind.

4. System nach einem der vorhergehenden Ansprüche, **dadurch gekennzeichnet, dass** der Draht (3) aus magnetischem Material mit transversaler Magnetisierung zur Drahtachse hergestellt ist.

5. System nach einem der vorhergehenden Ansprüche, **dadurch gekennzeichnet, dass** die magnetische Leistung des Drahtes (3) 0,2 bis 1 T beträgt.

6. System nach einem der vorhergehenden Ansprüche, **dadurch gekennzeichnet, dass** der Draht (3) hochflexibel ist.

7. System nach einem der vorhergehenden Ansprüche, **dadurch gekennzeichnet, dass** die Länge des Drahtes (3) im Bereich von 3 bis 30 cm liegt und eine bevorzugte Dicke des Drahtes (3) 1 bis 3 mm beträgt.

8. System nach einem der vorhergehenden Ansprüche, **dadurch gekennzeichnet, dass** der Magnetkern (4) des Drahtes (3) aus Magnetpulver besteht.

9. System nach einem der vorhergehenden Ansprüche, **dadurch gekennzeichnet, dass** der Magnetkern (4) des Drahtes (3) in einer flexiblen nichtmagnetischen Abdeckung (5) aus sterilem Kunststoff angeordnet ist.

10. System nach einem der vorhergehenden Ansprüche, **dadurch gekennzeichnet, dass** das System zur Isolierung zirkulierender Tumorzellen, hämatopoietischer Zellen, mesenchymaler Stammzellen oder anderer beliebiger zirkulierender Zellen in Abhängigkeit von den verwendeten monoklonalen Antikörpern geeignet ist.

## Revendications

1. Système d'isolement de cellules circulantes provenant de sang périphérique comprenant :
- une canule veineuse (1) adaptée pour injecter lentement les nanoparticules dans une veine d'un patient,
- des nanoparticules magnétiques recouvertes d'anticorps monoclonaux,
- des aimants (2) situés à l'extérieur du corps du patient et adaptés pour ajuster la position des nanoparticules injectées dans la veine,
- les aimants (2) étant en outre adaptés pour rapprocher les nanoparticules de la canule veineuse (1) avec le mouvement des aimants (2) après exposition des nanoparticules aux cellules sanguines circulantes et après la capture des cellules sanguines par liaison aux anticorps à la surface des nanoparticules,
- un fil (3) avec un noyau magnétique (4) adapté pour être inséré dans la veine à travers la canule (1),
- le fil (3) étant en outre adapté pour attirer les nanoparticules après le retrait ou la mise hors circuit des aimants (2), et
- un tube (6) avec un milieu adapté pour recevoir le fil (3) avec des cellules sanguines après son retrait de la canule (1) et dans lequel tube (6) les nanoparticules avec les cellules sanguines liées sont libérées après le retrait du noyau magnétique (4) du fil (3).

2. Système selon la revendication 1, **caractérisé en ce que** la canule veineuse (1) est une canule périphérique ou un cathéter veineux central.

3. Système selon la revendication 1 ou 2, **caractérisé en ce que** les aimants (2) ajustant la position des nanoparticules sont des électroaimants stationnaires ou des aimants permanents d'une intensité de 0,2 à 1 T.

4. Système selon l'une des revendications précédentes, **caractérisé en ce que** le fil (3) se compose d'un matériau magnétique avec une magnétisation transversale vers l'axe du fil.

5. Système selon l'une des revendications précédentes, **caractérisé en ce que** la puissance magnétique du fil (3) est de 0,2 à 1 T.

6. Système selon l'une des revendications précédentes, **caractérisé en ce que** le fil (3) est très souple.

7. Système selon l'une des revendications précédentes, **caractérisé en ce que** la longueur du fil (3) est comprise dans la plage allant de 3 à 30 cm et une épaisseur préférée du fil (3) est de 1 à 3 mm.

8. Système selon l'une des revendications précédentes, **caractérisé en ce que** le noyau magnétique (4) du fil (3) se compose d'une poudre magnétique.

9. Système selon l'une des revendications précédentes, **caractérisé en ce que** le noyau magnétique (4) du fil (3) est placé dans un revêtement non magnétique souple (5) en plastique stérile.

10. Système selon l'une des revendications précédentes, **caractérisé en ce que** le système est adapté pour l'isolement de cellules tumorales circulantes, de cellules hématopoïétiques, de cellules souches mésenchymateuses, ou de toute autre cellule circulante en fonction des anticorps monoclonaux utilisés.
